# EUROPEAN PATENT SPECIFICATION

(11) **EP 1 383 787 B1**
(45) Date of publication and mention of the grant of the patent: **03.12.2008**
(21) Application number: 02708534.9
(22) Date of filing: 28.03.2002
(51) Int. Cl.: C07J 71/00

(54) **SAPOGENIN DERIVATIVES, THEIR SYNTHESIS AND USE**
SAPOGENIN DERIVATE, DEREN SYNTHESE UND VERWENDUNG
DERIVES DE SAPOGENINE, SYNTHESE ET UTILISATION CORRESPONDANTES ET PROCEDES FONDES SUR LEUR UTILISATION

(30) Priority: 28.03.2001 GB 0107822; 27.03.2002 AR 0201170
(43) Date of publication of application: 28.01.2004
(73) Proprietor: PHYTOPHARM PLC, Godmanchester, Cambridgeshire PE29 2HY (GB)
(72) Inventor: BARRACLOUGH, Paul, Maidstone, Kent ME15 9SB (GB); HANSON, Jim, West Sussex BN44 3PF (GB); GUNNING, Phil, Cambs CB3 9NP (GB); REES, Daryl, Sandy SG19 2DD (GB); XIA, Zongqin, Shanghai 200025 (CN); HU, Yaer, Sanghai 200025 (CN)
(74) Representative: Brown, David Leslie
(86) International application number: PCT/GB2002/001578
(87) International publication number: WO 2002/079221

(56) References cited:
- WO-A-01/23406
- WO-A-99/48507
- DE-A- 4 303 214
- GIRAL F ET AL: "Esters of diosgenin and smilagenin" CHEMICAL ABSTRACTS + INDEXES, AMERICAN CHEMICAL SOCIETY. COLUMBUS, US, vol. 64, no. 6, 14 March 1966 (1966-03-14), XP002157709 ISSN: 0009-2258
- THOMPSON, M. J., ET. AL.: "The cholegenins. I. 16,22-epoxycoprostane-3 alpha,26,27-triol and its non-identity with dihydrocholegenin." J. AM. CHEM. SOC., vol. 81, 1959, pages 5225-5230, XP001074393
- MARKER, R.E., ROHRMANN, E.: "Sterols III. The structure of the Side Chain of Sarsasapogenin." J. AMER. CHEM. SOC., vol. 61, 1939, pages 846-851, XP001109547
- MARKER, R.E.,ROHRMANN, E., JONES, E.M.: "Sterols. XCIII. epi-pseudosarsasapogenin, pseudosarsasapogenone and pseudochlorogenin." J. AMER. CHEM. SOC., vol. 62, 1940, pages 648-649, XP002157703
- ZIEGLER, J.B., ET AL.: "The stereochemistry of steroidal sapogenins II." J. AMER. CHEM. SOC., vol. 77, 1955, pages 1223-1228, XP001109550
- CALLOW, ET AL.: "Epimerization at C(25) of steroid sapogenins: sarsasapogenin, neotigogenin and sisalagenin." J. CHEM. SOC., 1955, pages 1671-1674, XP001109548
- DATABASE WPI Derwent Publications Ltd., London, GB; AN 1993-339641 XP002157713 & JP 05 246866 A (KATO, K)
- YI N ET AL: "Sarsasapogenin: Mechanism in treating senile dementia" SYNTHESIS AND APPLICATIONS OF ISOTOPICALLY LABELLED COMPOUNDS, XX, XX, 14 September 1997 (1997-09-14), pages 315-320, XP002117140 cited in the application
- SHI, J., ET AL.: "The regulation effect of sarsasapogenin on M2 receptor density in CHO M2 cell" ACTA UNIVERSITATIS MEDICINALIS SECONDAE SHANGHAI, vol. 20, no. 6, - 2000 pages 503-505, XP008007424
- YI NINGYU ET AL: "Extraction of.beta.-adrenergic receptor- and M cholinergic receptor-regulating (.beta.,5.beta.,25s)-spirostan-3-ol from Anemarrhena asphodeloides Bunge" HCAPLUS, XP002168420

## Description

### Field of the Invention

The present invention relates to sapogenins and their derivatives, and enables their synthesis and use, and methods based upon their use.

A use of the sapogenins and their derivatives enabled by the present invention is in the treatment of cognitive dysfunction, non-cognitive neurodegeneration, non-cognitive neuromuscular degeneration, and receptor loss. The invention enables compositions for use in such treatments.

### Background of the Invention

Cognitive dysfunction is a characteristic of dementia conditions and syndromes, such as Alzheimer's disease (AD), senile dementia of the Alzheimer's type (SDAT), Lewi body dementia and vascular dementia. A lesser degree of cognitive dysfunction is also a characteristic of certain non-dementia conditions and syndromes, such as mild cognitive impairment (MCI), age-associated memory impairment (AAMI) and autism.

Non-cognitive neurodegeneration (i.e. neurodegeneration in the absence of cognitive dysfunction) and non-cognitive neuromuscular degeneration (i.e. neuromuscular degeneration in the absence of cognitive dysfunction) is a characteristic of conditions and syndromes such as Parkinson's disease, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, amyotrophic lateral sclerosis (ALS) and multiple sclerosis.

Receptor loss - particularly loss of nicotinic and/or muscarinic receptors and/or dopamine receptors and/or adrenoceptors - is a characteristic of some or all of the above conditions and syndromes. Said receptor loss in the absence of cognitive, neural and neuromuscular impairment is also a characteristic of conditions and syndromes such as postural hypotension, chronic fatigue syndrome, asthma, susceptibility to heart failure and macular degeneration.

The above conditions and syndromes are grave and growing problems in all societies where, because of an increase in life expectancy and control of adventitious disease, the demographic profile is increasingly extending towards a more aged population. Agents which can treat, or help in the management or prevention of such disorders, are urgently required.

WO-A-01/23406, claims, amongst other compounds, sapogenin derivatives of general formula (I): 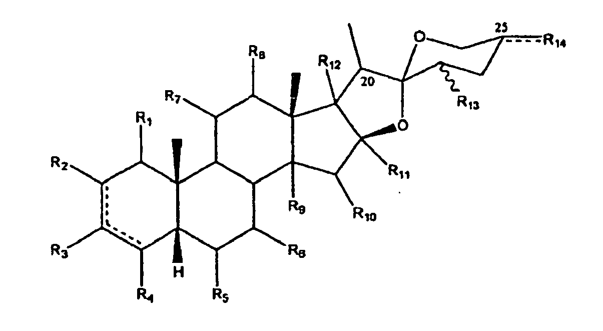 and their stereoisomers and racemic mixtures, their pharmaceutically acceptable pro-drugs and salts, wherein:
- R₁, R₂, R₃, R₄, R₅, R₆, R₇, R₈, R₁₀, are, independently of each other, either H, OH, =O, and OR where R = optionally substituted alkyl, optionally substituted acyl, optionally substituted carbamoyl, alkoxycarbonyl;
- R₉, R₁₂, R₁₁, R₁₃ can be either a H, OH, OR where R = optionally substituted alkyl, optionally substituted acyl, optionally substituted carbamoyl, alkoxycarbonyl;
- R₁₄ = optionally substituted alkyl group,
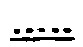 represents an optional double bond,
but excluding where simultaneously:
- R₁= R₂= R₄= R₅= R₆= R₇= R₈= R₁₀=R₁₁= R₉= R₁₂= R₁₃= H,
- R₃ = βOH,
- R₁₄ = CH₃
- the methyl group at C22 is α,
- the C20 is α, and there is a S configuration at C25;
and the use of these compounds as agents for increasing the muscarinic receptor number or enhancing the function of muscarinic receptors in a human or non-human animal, more particularly to treat cognitive dysfunction in diseases, more particularly still for the treatment of cognitive dysfunction in a patient suffering from a disease selected from AD, SDAT, Parkinson's disease, Lewi body dementia, autism, Myasthenia Gravis, Lambert Eaton disease, postural hypotension, chronic fatigue syndrome and diseases and problem associated with ageing.

According to the definitions contained in the description of WO-A-01/23406, in the variable groups of the above formula (I):
"Acyl" means an H-CO- or Alkyl-CO- group wherein the alkyl group is as defined below. Preferred acyls contain a lower alkyl. Exemplary acyl groups include formyl, acetyl, propanoyl, 2-methylpropanoyl, butanoyl and palmitoyl;
"Alkyl" means an aliphatic hydrocarbon group which may be straight or branched having about 1 to about 20 carbon atoms in the chain. Preferred alkyl groups have 1 to about 12 carbon atoms in the chain. Branched means that one or more lower alkyl groups such as methyl, ethyl or propyl are attached to a linear alkyl chain. "Lower alkyl" means about 1 to about 4 carbon atoms in the chain which may be straight or branched. Exemplary alkyl groups include methyl, ethyl, *n*-propyl, *i*-propyl, *n*-butyl, *t*-butyl, *n*-pentyl, 3-pentyl;
"Optionally substituted" means that the said group may be substituted with one or more substituents which may be the same or different, and include halo, alkyl, cycloalkyl, hydroxy, alkoxy, amino, acylamino, aryl, aroylamino, carboxy, alkoxycarbonyl, aralkoxycarbonyl, heteroaralkoxycarbonyl, optionally substituted carbamoyl;
"Pharmaceutically acceptable" means it is, within the scope of sound medical and veterinary judgement, suitable for use in contact with the cells-of humans and lower animals without undue toxicity, irritation, allergic response and the like, and are commensurate with a reasonable benefit/risk ratio. "Pharmaceutically acceptable prodrugs" means those prodrugs of the compounds which are, within the scope of sound medical and veterinary judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds. The term "prodrug" means compounds that are rapidly transformed *in vivo* to yield the parent compound of the above formula, for example by hydrolysis in blood. Functional groups which may be rapidly transformed, by metabolic cleavage, in vivo form a class of groups reactive with the carboxyl group. Because of the ease with which the metabolically cleavable groups of the compounds are cleaved in vivo, the compounds bearing such groups act as pro-drugs. A thorough discussion of prodrugs is provided in the following: Design of Prodrugs, H. Bundgaard, ed., Elsevier, 1985; Methods in Enzymology, K. Widder et al, Ed., Academic Press, 42, p.309-396, 1985; A Textbook of Drug Design and Development, Krogsgaard-Larsen and H. Bundgaard, ed., Chapter 5; Design and Applications of Prodrugs p.113-191, 1991; Advanced Drug Delivery Reviews, H. Bundgard, 8, p.1-38, 1992; Journal of Pharmaceutical Sciences, 77, p. 285, 1988; Chem. Pharm. Bull., N. Nakeya et al, 32, p. 692, 1984; Pro-drugs as Novel Delivery Systems, T. Higuchi and V. Stella, Vol. 14 of the A.C.S. Symposium Series, and Bioreversible Carriers in Drug Design, Edward B. Roche, ed., American Pharmaceutical Association and Pergamon Press, 1987.
"Pharmaceutically acceptable salts" means the relatively non-toxic, inorganic and organic acid addition salts, and base addition salts, of the compounds. These salts can be prepared in *situ* during the final isolation and purification of the compounds. In particular, acid addition salts can be prepared by separately reacting the purified compound in its free base form with a suitable organic or inorganic acid and isolating the salt thus formed. See, for example S. M. Berge, et al., Pharmaceutical Salts, J. Pharm. Sci., 66: p.1-19 (1977). Base addition salts can also be prepared by separately reacting the purified compound in its acid form with a suitable organic or inorganic base and isolating the salt thus formed. Base addition salts include pharmaceutically acceptable metal and amine salts.

According to the description in WO-A-01/23406, the effectiveness of the sapogenins of general formula I, including their stereoisomers and racemic mixtures, their pharmaceutically acceptable pro-drugs and salts is attributed at least in part to an activity of the compounds to normalise receptor number, i.e. to prevent decline in receptor number with time and also to restore receptor number from a depressed number to normal levels (page 20, lines 6 to 9).

DE-A-4303214 describes the use of a very wide range of saponins and sapogenins in the treatment of a wide range of viral diseases, but with no data that would allow one skilled in the art to select a preferred compound for any particular viral disease. Although Alzheimer's disease and Parkinson's disease are mentioned, these conditions are known to be of non-viral origin, with the result that no relevant teaching can be discerned in the document.

WO-A-99/16786 (published 8 April 1999) describes the use of natural saponins for the treatment of dementia. Saponins tend to be water-soluble, whereas sapogenins are lipid-soluble and therefore saponins are less effective in crossing the blood-brain barrier.

Chinese Patent Application No. CN-A-1096031 describes the use of the spirostane sapogenin, sarsasapogenin, in the two-way regulation of β-adrenergic and M-cholinergic receptors. No specific pharmaceutical activity is suggested. However, in "Synthesis and Applications of Isotopically Labelled Compounds", 1998, pages 315-320, Yi et al describe the use of sarsasapogenin in the treatment of senile dementia.

WO 99/48507 discloses the use of various sapogenins, such as smilagenin and sarsasapogenin, in the treatment of cognitive dysfunction and allied conditions.

In the cases of Parkinson's disease, myasthenia gravis, Lambert Eaton disease, postural hypotension and chronic fatigue syndrome, however, cognitive dysfunction is not a primary symptom, although it may be present as one of a number of possible secondary symptoms. Moreover, these conditions are not viral diseases or dementias. Many of these disorders are so-called "spectrum" disorders, in which a wide range of combinations of symptoms, in a wide range of relative severities, present themselves. Therefore, in many instances, a treatment for cognitive dysfunction (e.g. dementia) is not necessary.

The present invention is based upon our finding that certain sapogenins and their derivatives, including compounds from within the formula I as defined in WO-A-01/23406, have a surprising activity against non-cognitive neurodegeneration and non-cognitive neuromuscular degeneration, as well as against receptor loss in the absence of cognitive, neural and neuromuscular impairment. This finding enables improved treatment of certain non-viral spectrum and non-spectrum disorders in which cognitive dysfunction is not a primary symptom, such as, for example, Parkinson's disease, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, amyotrophic lateral sclerosis (ALS), multiple sclerosis, postural hypotension, chronic fatigue syndrome, asthma, susceptibility to heart failure, and macular degeneration.

In addition, we have found that certain of the compounds have activity against cognitive dysfunction, which was not previously disclosed.

### Brief Description of the Invention

In one aspect of the invention, there is provided compounds of the general formula II: 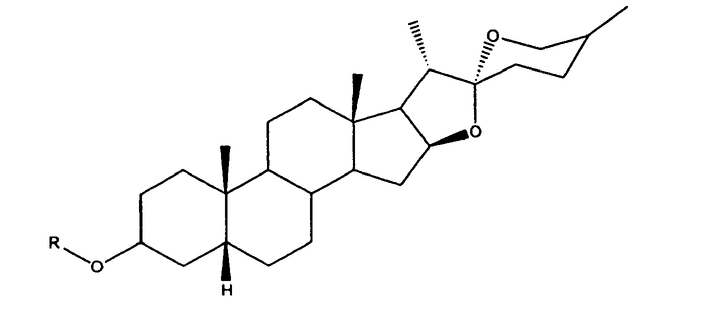 wherein the group R is selected from (C₁₋₄ alkyl)carbonyl and (C₁₋₄ alkoxy)carbonyl, optionally substituted with a terminal carboxylic acid residue (-COOH);
provided that:
R is not unsubstituted acetyl;
R is not unsubstituted ethoxycarbonyl when simultaneously the stereochemistry of C3 is S(β) and of C25 is R;
R is not succinyl when simultaneously the stereochemistry of C3 is S(β) and of C25 is
S or the stereochemistry of C3 is R(α) or S(β) and of C25 is R; and
R is not propionyl, butyryl, isobutyryl, valeryl or isovaleryl when the stereochemistry of C25 is R and the stereochemistry of C3 is S(β);
and pharmaceutically acceptable salts thereof.

In another aspect of the invention, there is provided compounds of formula II as defined above for use in the treatment of one of: Parkinson's disease, postural hypotension, autism, chronic fatigue syndrome, Myasthenia Gravis, Lambert Eaton disease, Huntington disease, multiple sclerosis, asthma, heart failure and epilepsy.

In a further aspect of the invention, there is provided a pharmaceutical composition which comprises a pharmacologically effective amount of a compound of formula II as defined above.

In yet another aspect of the invention, there is provided a foodstuff or beverage which comprises a pharmacologically effective amount of a compound of formula II as defined above.

The present invention enables the treatment of human and non-human animals suffering from any of: Parkinson's disease, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, amyotrophic lateral sclerosis (ALS), multiple sclerosis, postural hypotension, chronic fatigue syndrome, asthma, susceptibility to heart failure, and macular degeneration.

The invention enables the use of the compounds of formula II and their pharmaceutically acceptable salts,
in the treatment or prevention of, or in the preparation of compositions (e.g. pharmaceutical compositions, foodstuffs, food supplements and beverages) for the treatment or prevention of, (i) cognitive dysfunction, (ii) non-cognitive neurodegeneration, (iii) non-cognitive neuromuscular degeneration, or (iv) receptor loss in the absence of cognitive, neural and neuromuscular impairment, in human and non-human animals suffering therefrom or susceptible thereto.

In one aspect the C₂₅ methyl group is in the S configuration; these compounds of the invention are sarsasapogenin and episarsasapogenin or derivatives thereof. In another aspect, the C₂₅ methyl group is in the R configuration; these compounds of the invention are smilagenin and epismilagenin or derivatives thereof.

The invention also enables compositions containing the active agents for use in the said treatment methods, and methods for the preparation of the active agents. These are discussed in more detail below

The active agents of the invention may, if desired, be co-administered with one or more additional active agents for example cholinesterase inhibitors and L-dopa.

### Detailed Description of the Invention

### Active Agents

In the above definition of formula II:
Optional -COOH substituents of alkyl groups, where present, are at the terminal position of the alkyl group.
"Alkyl" means an aliphatic hydrocarbon group which may be straight or branched having 1 to 4 carbon atoms in the chain. Branched means that one or more lower alkyl groups such as methyl or ethyl are attached to a linear alkyl chain. "Lower alkyl" means 1 to 4 carbon atoms in the chain which may be straight or branched. Exemplary alkyl groups include methyl, ethyl, *n*-propyl, *i*-propyl, *n*-butyl, *t*-butyl and *s*-butyl.
"Carboxylic acid residue" means the group -COOH.
"Pharmaceutically acceptable salts" means the relatively non-toxic, inorganic and organic acid addition salts, and base addition salts, of compounds of the present invention. These salts can be prepared *in situ* during the final isolation and purification of the compounds. In particular, acid addition salts can be prepared by separately reacting the purified compound in its free base form with a suitable organic or inorganic acid and isolating the salt thus formed. See, for example S. M. Berge, et al., Pharmaceutical Salts, J. Pharm. Sci., 66: p.1-19 (1977). Base addition salts can also be prepared by separately reacting the purified compound in its acid form with a suitable organic or inorganic base and isolating the salt thus formed. Base addition salts include pharmaceutically acceptable metal and amine salts. Examples of suitable acid addition salts are those formed with acids selected from hydrochloric, sulphuric, phosphoric and nitric acids. Examples of suitable base addition salts are those formed with bases selected from sodium hydroxide, potassium hydroxide and ammonium hydroxide.
"Pharmaceutically acceptable" means that the material is, within the scope of sound medical and veterinary judgement, suitable for use in contact with the cells of humans and lower animals without undue toxicity, irritation, allergic response and the like, and is commensurate with a reasonable benefit/risk ratio.

In the above formula II, -OR may, for example, be selected from the following (unless excluded by proviso): cathylate (ethoxycarbonyloxy), acetate, succinate, propionate, butyrate, valerate and isovalerate.

In the above formula II, the group R is selected from lower alkyl and lower alkoxy, optionally substituted with a terminal carboxylic acid (-COOH) residue.

Of the compounds of general formula II and their pharmaceutically acceptable salts, particularly preferred are the following compounds (unless excluded by proviso):
sarsasapogenin cathylate
episarsasapogenin cathylate
episarsasapogenin succinate and pharmaceutically acceptable salts thereof
smilagenin cathylate
epismilagenin cathylate.

A particularly preferred active agent is episarsasapogenin, its cathylate, and succinate, esters, and pharmaceutically acceptable salts thereof.

The active agents may be formulated for delivery as pharmaceutically acceptable prodrugs, which term shall be understood in the same way as defined in WO-A-01/23406, referred to above.

### Compositions and Uses

The present invention, enables the use of the inventive compounds for treating or preventing non-cognitive neurodegeneration, non-cognitive neuromuscular degeneration, or receptor loss in the absence of cognitive, neural or neuromuscular impairment, in a human or non-human animal in need thereof, whereby an effective dosage of a compound of general formula II as defined above or a pharmaceutically acceptable salt thereof is to be administered to said human or non-human animal.

The present invention, also enables the use of the inventive compounds for treating or preventing cognitive dysfunction in a human or non-human animal in need thereof, whereby an effective dosage of a compound of general formula II as defined above or a pharmaceutically acceptable salt thereof is to be administered to said human or non-human animal.

The active agent may be administered in the form of a composition comprising the active agent and any suitable additional component. The composition may, for example, be a pharmaceutical composition (medicament), a foodstuff, food supplement or beverage. Such a composition may contain a mixture of the specified compounds, and/or of their pharmaceutically acceptable salts.

The present invention, further enables a composition having activity against non-cognitive neurodegeneration, non-cognitive neuromuscular degeneration, or receptor loss in the absence of cognitive, neural or neuromuscular impairment, in a human or non-human animal, which comprises an effective amount of a compound of general formula II as defined above or a pharmaceutically acceptable salt thereof.

The present invention, also enables a composition having activity against cognitive dysfunction in a human or non-human animal, which comprises an effective dosage of a compound of general formula II as defined above or a pharmaceutically acceptable salt thereof.

The term "pharmaceutical composition" in the context of this invention means a composition comprising an active agent and comprising additionally pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms.

The terms "foodstuff", "food supplement" and "beverage" used herein have the normal meanings for those terms, and are not restricted to pharmaceutical preparations.

The dosage of the active agent will vary widely, depending on the severity of the symptoms to be treated or prevented. The selection of appropriate dosages is within the ability of one of ordinary skill in this art, without undue burden. The dosage of the active agent may, for example, be greater than about 0.3 mg/kg body weight, preferably administered once per day. More typically, the dosage will be between about 1 and about 25 mg/kg, e.g. between about 1 and about 10 mg/kg, preferably administered once per day. The compositions may suitably be formulated as unit dosage forms, adapted to administer a unit dosage of between about I and about 10 mg/kg to the patient, the number and frequency of administrations in a particular time period to be as instructed. For human use, the dosage may conveniently be between about 70 and about 700 mg per day.

"Pharmaceutically acceptable dosage forms" means dosage forms of the compounds or compositions of the invention, and includes, for example, tablets, dragees, powders, elixirs, syrups, liquid preparations, including suspensions, sprays, inhalants tablets, lozenges, emulsions, solutions, granules, capsules and suppositories, as well as liquid preparations for injections, including liposome preparations. Techniques and formulations generally may be found in Remington, Pharmaceutical Sciences, Mack Publishing Co., Easton, PA, latest edition.

In general, reference herein to the presence of one of a specified group of compounds includes within its scope the presence of a mixture of two or more of such compounds.

This invention enables the use of the inventive compounds for the treatment of cognitive dysfunction in a patient suffering from one of: Alzheimer's disease, SDAT, AAMI, Lewi body dementia or autism, whereby a pharmacologically effective amount of a compound of formula II or of a pharmaceutically acceptable salt thereof is to be administered to the patient.

This invention, also enables the use of the inventive compounds for enhancing cognitive function in a human or non-human animal, whereby an effective amount of a compound of formula II or of a pharmaceutically acceptable salt thereof is to be administered to the patient. The treatment may be a non-therapeutic method practiced on a normal subject, for enhancing the subject's cognitive function.

This invention, further enables the use of the inventive compounds for the treatment of (i) non-cognitive neurodegeneration, (ii) non-cognitive neuromuscular degeneration, or (iii) receptor loss in the absence of cognitive, neural or neuromuscular impairment, in a human or non-human animal in a patient suffering from one of: Parkinson's disease, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, amyotrophic lateral sclerosis (ALS), multiple sclerosis, postural hypotension, chronic fatigue syndrome, asthma, susceptibility to heart failure, and macular degeneration, whereby a pharmacologically effective amount of a compound of formula II or of a pharmaceutically acceptable salt thereof is to be administered to the patient.

The uses for enhancing cognitive or neurological function and the uses for treating certain conditions, as defined above, may be accomplished by administering the compound or composition or medicament, as the case may be, in the form of a pharmaceutical composition, foodstuff, food supplement or beverage.

The invention also enables the use of one or more compounds of formula II or of a pharmaceutically acceptable salt thereof as an ingredient in a pharmaceutical composition, food product, food supplement or beverage in a method for the treatment of Parkinson's disease, muscular dystrophy including facioscapulohumeral muscular dystrophy (FSH), Duchenne muscular dystrophy, Becker muscular dystrophy and Bruce's muscular dystrophy, Fuchs' dystrophy, myotonic dystrophy, corneal dystrophy, reflex sympathetic dystrophy syndrome (RSDSA), neurovascular dystrophy, myasthenia gravis, Lambert Eaton disease, Huntington's disease, amyotrophic lateral sclerosis (ALS), multiple sclerosis, postural hypotension, chronic fatigue syndrome, asthma, susceptibility to heart failure, and macular degeneration.

The invention also enables the use of one or more compounds of formula II or of a pharmaceutically acceptable salt thereof, as an ingredient in a pharmaceutical composition, food product, food supplement or beverage in the treatment of Alzheimer's disease, SDAT, AAMI, MCI and autism.

### Preparation of compounds for Use in the Invention

Smilagenin, epismilagenin, sarsasapogenin and episarsasapogenin are commercially available materials. Suppliers include, for example, Sigma Aldrich, Research Plus Inc. and Steraloids Inc. Preparative methods for these materials are also to be found in the literature (e.g. a preparation of episarsasapogenin is given in JACS p.5225 (1959)). Episarsasapogenin can be prepared by reduction of sarsasapogenone using a metal hydride reducing agent. Sarsasapogenone can be prepared using the method of Lajis et al, Steroids, 1993, 58, 387-389.

Also, as starting materials, unsubstituted sapogenins may occur naturally in a range of plant species, notably plants of the genus Smilax, Asparagus, Anemarrhena, Yucca or Agave. Where smilagenin or sarsasapogenin is used, it may be in the form of a plant extract, or dry powdered plant material, derived from a plant of the genus Smilax, Asparagus, Anemarrhena, Yucca or Agave.

The compounds of formula II can be prepared using conventional techniques from compounds in which R = H. The preferred reaction is a nucleophilic substitution reaction, in which a compound of formula 11 in which R = H is reacted with a compound of formula

L-R,

in which R is selected from (C₁₋₄ alkyl) carbonyl; or (C₁₋₄ alkoxy) carbonyl; wherein any alkyl group is optionally substituted with a terminal carboxylic acid residue (-COOH); and L is a leaving group, under conditions suitable for nucleophilic substitution.

The compound L-R may, for example, be a carboxylic acid or, if appropriate, an anhydride, or an acyl halide (e.g. an acyl chloride). For example, where R is a cathylate (ethoxycarbonyl) moiety, the compound L-R may suitably be ethyl chloroformate.

The reaction is suitably performed in a base such as pyridine, optionally in the presence of an acid such as hydrochloric acid.

The reaction details for nucleophilic substitution reactions are well known. See, for example, RC Larock, in Comprehensive Organic Transformations, VCH publishers, 1989.

In the reactions described herein it may be necessary to protect reactive functional groups, for example hydroxy, carboxy or amino groups, where these are desired in the final product, to avoid their unwanted participation in the reactions. Conventional protecting groups may be used in accordance with standard practice. For examples, see TW Green and PGM Wuts, in "Protective Groups in Organic Chemistry", John Wiley & Sons, 1991; JFW McOmie in "Protective Groups in Organic Chemistry", Plenum Press, 1973.

The compound thus prepared may be recovered from the reaction mixture by conventional means. For example, the compound may be recovered by distilling off the solvent from the reaction mixture or, if necessary after distilling off the solvent from the reaction mixture, pouring the residue into water, followed by extraction with a water-miscible solvent and distilling off the solvent from the extract. Additionally, the product can, if desired, be further purified by various well known techniques, such as recrystallisation, reprecipitation, or the various chromatography techniques, notably column chromatography or preparative thin layer chromatography.

### Novel Compounds

The compounds of general formula II, and their pharmaceutically acceptable salts, are new *per se*, and these novel compounds constitute an aspect of the present invention.

There may be mentioned particularly as novel compounds the compounds of formula II in which R is any of the stated groups except acetyl.

Novel salts of the compounds of general formula II, including novel salts of compounds of general formula II which are not themselves pharmaceutically acceptable, may find use as intermediates in methods for the preparation of the compounds of general formula II and their pharmaceutically acceptable salts.

### Discussion of the Basis for the Activity

Without wishing to be bound by theory, it is believed that the compounds defined above exhibit the ability to regulate receptors. For example, some of these compounds have been found to prevent or reverse the loss of muscarinic receptors or dopamine receptors in the brain. It is believed that the compounds function by rectifying a deficiency in receptor number or function or turnover in the animal being treated.

One hypothesis is that the compounds are increasing the synthesis or release of, or are decreasing the rate of degradation of, neurotropic factors such as brain derived growth factor and/or nerve growth factor. These effects on growth factors might be due to an effect of the compound on a cytosolic or nuclear receptor, or the binding of a compound to a promoter region with a consequent effect directly on the rate of production of mRNA for the growth factor, or as a consequence of increasing the production of another material factor.

The increased expression and/or abnormal processing of the amyloid precursor protein (APP) is associated with the formation of amyloid plaques and cerebrovascular amyloid deposits which are the major morphological hallmarks of Alzheimer's disease. Of particular interest are the processes regulating the proteolytic cleavage of APP into amyloidogenic and nonamyloidogenic fragments. The cleavage of APP by the enzyme α-secretase within the β-amyloid sequence of the protein results in the formation of a non amyloidogenic C-terminal fragment, and the soluble APPsα fragment; this latter fragment has been shown to have neurotropic and neuroprotective activity as well as to enhance memory in mice when injected intra-cerebro-ventrically (ICV). In contrast, processing of APP by β-secretase exposes the N-terminus of β-amyloid which is released by γ-secretase cleavage at the variable C-terminus. The resulting β-amyloid peptides, which contain 39-43 amino acids, have been shown to be neurotoxic and to accumulate in plaques which interfere with inter-neurone connections.

A number of studies have shown that stimulation of muscarinic receptors results in an increase in α-secretase activity. As a consequence processing of APP to APPsα with its neuroprotective effects is increased. In parallel, processing of APP by β- and γ-secretase is decreased and there is a consequential reduction of β-amyloid. Other transmitters such as nerve growth factor (NGF) and brain derived neurotropic factor (BDNF) as well as bradykinin and vasopressin may have similar effects in increasing the proportion of APP processed to APPsα. There may be a number of factors involved in the effects of NGF which may include binding of the factor to the tyrosine kinase receptor (TrkA) and the stimulation of phospholipase Cγ with subsequent phosphorylation and activation of protein kinase C (PKC) and increase in relative activity of α-secretase.

Compounds according to this invention which reverse the loss of, and/or increase, muscarinic receptor numbers will have particular utility. Indeed the benefits may be seen in three parts as follows.
1. An increase in muscarinic receptor numbers leading to increased synaptic transmission; the reversal of the loss of, and/or increase in, the number of nicotinic receptors, which lie upstream of the synaptic cleft, will lead to an increase in, or a reversal of loss of, acetylcholine release into the synaptic cleft, thereby increasing muscarinic receptor activation and thus amplifying the overall effect.
2. Secondary to the increased receptor numbers with a consequential increase in α-secretase activity, leading to:
   2.1 A reduced production of β-amyloid and a consequent reduction of plaque formation and neuronal loss;
   2.2 An increase in APPsα and a consequent improvement in cerebral function as witnessed by an improvement in short and long term memory.

### Brief Description of the Drawings

In order to illustrate the invention further by way of non-limiting example, reference will now be made to the accompanying drawings and to the Examples which follow.

In the drawings:
Figure 1 illustrates a hypothetical mode of action for the compounds employed in the uses enabled by this invention;
Figure 2 shows the effects of sarsasapogenin, episarsasapogenin cathylate and smilagenin on the learning ability and memory of aged rats;
Figure 3 shows the effects of sarsasapogenin, episarsasapogenin cathylate and smilagenin on muscarinic receptor number,
Figure 4 shows the effects of sarsasapogenin, episarsasapogenin cathylate and smilagenin on glutamate induced neurodegeneration in rat primary cortical neurons; and
Figure 5 shows the effect of epismilagenin acetate on m3 and β2 adrenoceptor density at day 5 in a CHO-β2/m3 co-transfected cell line.

### Detailed Description of the Drawings and Examples

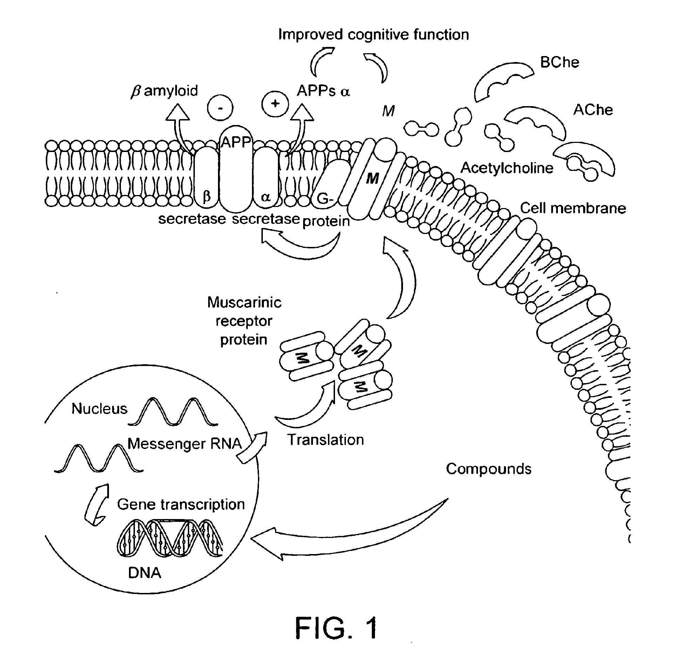

Referring to Figure 1 of the drawings, a diagrammatic representation of the function of the compounds of the invention is shown. It is believed that the compounds act primarily on cell nuclei; the invention is not, however, limited to any particular mode of action. The observed increase in receptor number consequential upon administration of an active agent is interpreted as leading to increased expression of muscarinic (and/or nicotinic and/or dopamine) receptor protein. The possible link between the secretases and β-amyloid protein formation (discussed above) is indicated in the drawing.

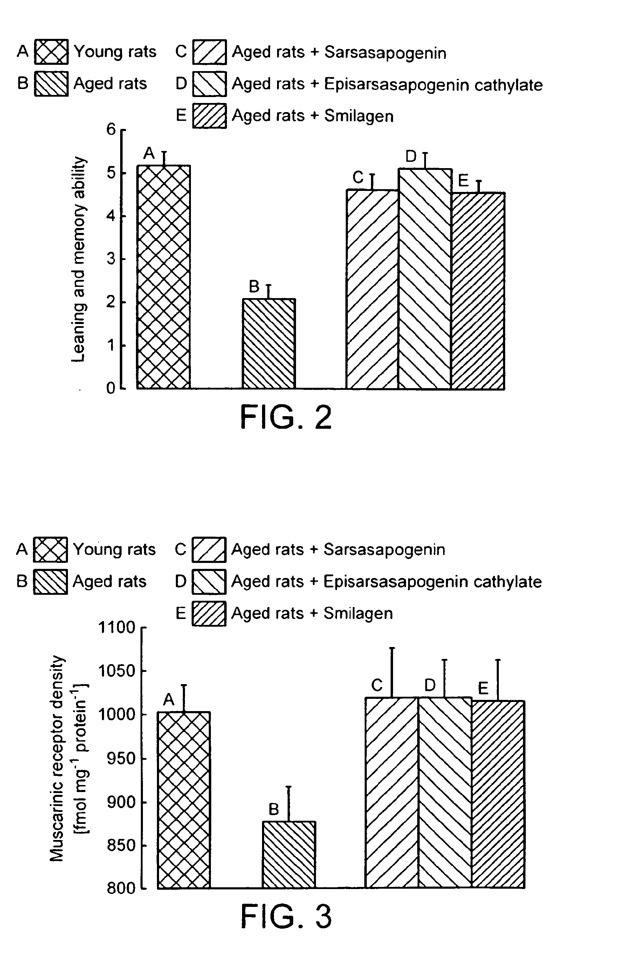

Figures 2 to 5 will be described in detail below, in connection with the discussion of the examples.

Epismilagenin cathylate, epismilagenin acetate, sarsasapogenin cathylate, episarsasapogenin cathylate, episarsasapogenin succinate, episarsasapogenin ethylsuccinate (comparison), sarsasapogenin, episarsaspogenin, smilagenin and epismilagenin have been tested for activity in a number of *in vitro* and *in vivo* assays. The assays/experiments that were considered of key importance in determining possible activity in the modulation of receptor numbers were as follows:

### Assay 1: Cell based assay

Chinese hamster ovary (CHO) cells transfected with a vector coding for a muscarinic receptor and/or beta adrenoreceptor. The cell line used for the majority of the experiments was a cell line expressing a muscarinic receptor.

### Assay 2: Alzheimer's disease model

An *in vivo* model of Alzheimer's disease in which amyloid beta and ibotenic acid are injected into the brain of the rat.

### Assay 3: Learning and memory test

A Y-maze used to test learning and memory in rats exposed to the test compounds. The rats were subsequently sacrificed and the density of muscarinic receptors in the brain assayed by dual-site competitive binding assay, to correlate performance in the Y-maze, receptor density and activity of the active agents.

### Assay 4: Neuroprotection of cultured neurons

An *in vitro* test of the ability of the test compounds to protect neurons against damage in an environment hostile to neurons.

### Methods and Results

The methods and the results of these experiments are now described in the following Examples, which also give examples of methods of synthesis.

### EXAMPLE 1

### Cell based assay

The effects of epismilagenin cathylate*, sarsasapogenin cathylate*, episarsasapogenin cathylate*, episarsasapogenin succinate*, epismilagenin acetate** and sarsasapogenin** on the expression of m receptors in CHO cells transfected with vector for the m receptor were investigated. Receptor numbers were assayed using [³H]NMS binding and subtracting non-specific binding. Compounds were dissolved in dimethyl sulphoxide (DMSO) and DMSO was used as a control.
* Compounds in accordance with the present invention.
** Compounds assisting understanding of the present invention.

### Methods:

Chinese hamster ovary (CHO) cells expressing high levels of muscarinic receptor (~2.2 pmol mg protein⁻¹) were plated on a 24 well plate, 1 day before the start of the experiment. The culture medium was replaced with medium containing vehicle (DMSO) or the compounds. The cells were incubated for 2/3 days, then after a medium change, cells were incubated for a further 2/3 days. The cells were incubated with a saturating concentration of labelled N-methyl-scopolamine, ([³H]NMS). Cells were washed with ice cold phosphate-buffered saline (PBS) (3x) and bound [³H]NMS determined by solubilising receptors with RIPA buffer followed by liquid scintillation counting.

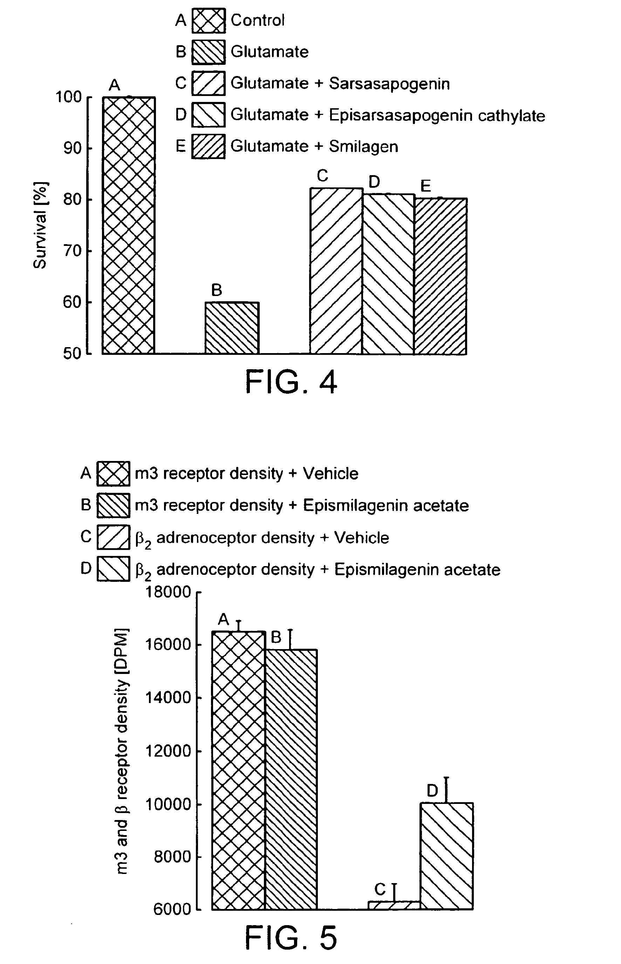

The results shown in Figure 5 used a CHO cell line co-transfected to express both β2 adrenoceptors and m3 muscarinic receptors. To measure the β2 and m3 receptor density, both [³H]NMS and [³H]CGP were used.

### Results:

These are illustrated in Table 1 below and in Figure 5 of the drawings. Over the culturing period, treatment with epismilagenin cathylate, sarsasapogenin cathylate, episarsasapogenin cathylate, episarsasapogenin succinate and sarsasapogenin each prevents the decrease in muscarinic receptor number. Co-incubation of the co-transfected cell line with epismilagenin acetate (Figure 5) did not significantly alter the density of m3 receptors,whereas the decrease in β2 adrenoceptors was significantly prevented by the epismilagenin acetate.

**Table 1**

| **Compound** | **Concentration [microMolar]** | **Activity** |
|---|---|---|
| epismilagenin cathylate | 10 | ++ |
| sarsasapogenin cathylate | 10 | ++ |
| episarsasapogenin cathylate | 10 | ++ |
| episarsasapogenin succinate | 10 | ++ |
| Sarsasapogenin | 10 | ++ |

Thus, the experiments indicate that each of epismilagenin cathylate, sarsasapogenin cathylate, episarsasapogenin cathylate, episarsasapogenin succinate, epismilagenin acetate and sarsasapogenin were able to increase the number of muscarinic receptors or adrenoceptors expressed in CHO cells cultured in vitro. The compounds of this invention act to normalise receptor number i.e. they tend to prevent decline in receptor number with time and also tend to restore receptor number to normal levels when given to cells in which the receptor level is depressed.

### EXAMPLE 2

### Alzheimer's disease model

An *in vivo* model of Alzheimer's disease was used in which amyloid beta and ibotenic acid are injected into the brain of the rat, which leads to a receptor loss in the brain and cognitive impairment. Previous studies showed that local injection of amyloid β in the nucleus vasalis of the rat brain caused cholinergic hypofunction and behavioural impairment up to two months post surgery (Giovannelli et al., 1995: Neuroscience, 66, 781-792.). In addition the co-injection of amyloid β with a small amount of ibotenic acid into the rat hippocampus synergistically produces neuronal loss with infiltration of glial cells not only adjacent but also far from the injected site (Morimoto et al., 1998: Neuroscience, 84, 479-487).

### Methods:

Our studies used the method of Morimoto (Morimoto et al., 1998: Neuroscience, 84, 479-487) with some modifications (unilateral instead of bilateral injection). Three months old, Sprague Dawley rats, were randomly divided into different groups. Injection of amyloid β₁₋₄₀ and ibotenic acid (both from Sigma) was accomplished by means of a stereotaxic instrument (Stoelting Co.) and the coordinates were AP= -0.5mm (right to medial line), L= -2.8mm (backward from bregma), H= -7.0mm (ventral to dura). The dose for each rat was amyloid β₁₋₄₀ (4 µg) and ibotenic acid (1 µg) in 1 µl of saline. The injection was completed in 20 min, and the needle was withdrawn 10 min later. Then the skin was sutured.

The 8 groups were:
Operated control injected with normal saline (control)
Model (control injected with amyloid β+ ibotenic acid)
Model + Episarsasapogenin cathylate (18 mg/kg/day)*
Model + Sarsasapogenin cathylate (18 mg/kg/day)*
Model + Episarsasapogenin ethylsuccinate (18 mg/kg/day) (comparison)
Model + Episarsasapogenin (18 mg/kg/day)**
Model + Epismilagenin (18 mg/kg/day)**
Model + Diosgenin (i.e. negative control, 18 mg/kg/day)
* Compounds in accordance with the present invention.
** Compounds assisting understanding of the present invention.

### Drug administration

Episarsasapogenin cathylate, sarsasapogenin cathylate, episarsasapogenin ethylsuccinate (comparison compound), episarsasapogenin, epismilagenin and diosgenin (all at a dosage of 18 mg/kg/day) were administered to animals as stable suspensions in CMC-Na (0.5%) once daily through a gastric tube. The control and the Alzheimer's model group were given the same volume of CMC-Na (0.5%) once daily. The drugs and vehicles were given for a period of two months, starting 20 days before operation.

### Measurement of muscarinic-receptor density

The brain samples were homogenised, centrifuged, and the pellet of centrifugation at 27000xg was re-homogenised and used for measurement. The concentration of ³H-QNB was chosen at the saturation range. After incubation and separation, the bound portion was measured by liquid scintillation counter.

The effect of test compounds on memory was assessed using the step-through test. A 60 x 15 x 15 cm box, divided into 2 equally sized rooms, one dark room with copper rod base, which was electrically charged (40 V ac) when in use, while the other was a light room but not electrically charged. Between the two rooms there is an opening (hole) for the rat to go through. The experiment is carried out for each rat on two consecutive days. The first day is for training; when the rat is adapted in the box for the first 3 min, then put in the light room, with its back toward the hole, and the copper rods of the dark room are charged for 5 min. The second day is for testing, when the number of crosses in 5 min are recorded. Improvements in memory are signalled by a reduction in the number of crosses.

### Results

The muscarinic receptor density in Alzheimer's model brains was significantly lower than control. Episarsasapogenin cathylate, sarsasapogenin cathylate, episarsasapogenin and epismilagenin produced a significant elevation in brain muscarinic receptor density, whereas diosgenin and episarsasapogenin ethylsuccinate did not significantly change the muscarinic receptor density. Thus the experiments indicate that the compounds of this invention act to normalise receptor number, i.e. they tend to restore receptor number to normal levels when given to animals in which the receptor level is depressed.

The number of wrong responses (error number) in 5 min was significantly higher in the Alzheimer's model group than the control group, indicating an impairment of memory (see Table 2 below). Epismilagenin, episarsasapogenin cathylate, episarsasapogenin and sarsasapogenin cathylate each significantly decreased the number of wrong responses, whereas diosgenin and episarsasapogenin ethylsuccinate were both ineffective in decreasing the number of wrong responses.

**Table 2**

| Group | Muscarinic receptor Density | Step through test |
|---|---|---|
| | (fmol/mg/protein) | Error No |
| Control (n=10) | 859±101 | 0.60±0.70 |
| Model (n=10) | 713±48 | 4.00±2.40 |
| Model + Episarsasapogenin cathylate | | |
| (n= 10) | 877±89* | 1.36±0.92* |
| Model + Sarsasapogenin cathylate | | |
| (n=11) | 916±158 * | 1.36±1.03* |
| Model + Episarsasapogenin | | |
| ethylsuccinate (n=11) | 774±79 | 3.73±1.35 |
| Episarsasapogenin (n=10) | 869±104* | 1.50±1.18* |
| Epismilagenin (n=11) | 877±90* | 1.73±0.91* |
| Model + Diosgenin (n=8) | 770±68 | 3.75±1.49 |

| | | |
|---|---|---|
| Statistical analysis using unpaired Student t test. * denotes p<0.05 | | |

### EXAMPLE 3

### Learning and memory test

Aged Sprague-Dawley rats aged were divided randomly into 4 groups, one control and groups treated for three months with either sarsasapogenin**, episarsasapogenin cathylate* or smilagenin** (18 mg kg⁻¹ day⁻¹, n = 10). A control group (n=14) of untreated young rats was also included in the study. The daily dose of drug was mixed in a minimum amount of food and was administered every morning separately to each rat.
* Compounds according to the invention.
** Compounds assisting understanding of the invention.

A Y-maze apparatus was used for the learning and memory test. On the floor of each arm of the Y-maze is an array of copper rods to which electric current is applied whenever needed, with adjustable voltage. Each arm is 45 cm long and has a 15 W lamp at the end, which is turned on when needed. After 3 months drug administration, each rat was trained for 7 consecutive days, as follows. For each training session, the rat was put into one arm of the Y-maze, after two minutes rest, an electrical current was applied to the copper rods and the lamp of the clockwise arm was illuminated to indicate the non-stimulation area. If the rat went into that arm, one correct response was recorded, otherwise, one wrong response was recorded. This stimulation-response test was repeated 20 times each day, with a pause of 5 sec between each two consecutive tests. The number of correct responses following the twenty tests on the seventh day was used to express learning ability, (the higher the number the better the learning ability). The rats were then left resting for 30 days and the procedure was repeated once more. The number of correct responses of the 20 tests after the 30 day rest period was used to represent the memory ability

### Measurement of muscarinic receptor density in the brain

Tissue preparation: Brains were removed quickly after decapitation, frozen in dry ice, and transferred to a freezer. The brains were homogenised and the pellet was finally suspended in buffer.

Dual-site competitive ligand binding assay: ³H-QNB (quinuclinidyl benzilate) was used as the radio-ligand which was non-selective to M receptor subtypes *in vitro.* Pirenzipine was used as the selective non-radioactive competing agent. Protein concentration was determined by the micro-Lowry method.

The results are shown in Figures 2 and 3 of the drawings. The Y-maze experiments revealed that both the learning ability and memory are impaired in aged rats. Sarsasapogenin, episarsasapogenin cathylate and smilagenin restored the learning and memory ability following administration in aged rats. Muscarinic receptor density was markedly reduced in aged rats. Sarsasapogenin, episarsasapogenin cathylate and smilagenin significantly restored the muscarinic receptor number.

### Conclusions

Sarsasapogenin, episarsasapogenin cathylate and smilagenin significantly restores the muscarinic receptor density in the aged rat brain toward that of young rats. The restoration of muscarinic receptor density in aged rat brain induced by sarsasapogenin, episarsasapogenin cathylate and smilagenin is associated with an increase in learning ability and memory.

### EXAMPLE 4

### Neuroprotective effect of sarsasapogenin, episarsasapogenin cathylate and smilagenin

The objective of this study was to examine the effects of sarsasapogenin**, episarsasapogenin cathylate* and smilagenin** on the survival of rat primary cortical cultures treated with glutamate, which is known to induce neurodegeneration.
* Compounds according to the invention.
** Compounds assisting the understanding of the invention.

### Primary cultures of cortical neurons

Rat cortical neurons were cultured for 10 days; at day 10 the medium was changed to a serum-free defined medium. On day 12, 24 hours before glutamate exposure, cultures were washed and medium was replaced with fresh medium containing positive control (β-oestradiol), test compounds (sarsasapogenin, episarsasapogenin cathylate or smilagenin) or vehicle control (DMSO, 0.25%).

On day 13, cultures were exposed to glutamate.

After the incubation period, the cultures were washed with and placed in fresh medium, supplemented with relevant compounds or vehicle to evaluate their protective effects, 24 h after glutamate exposure.

Neuronal cell survival was evaluated by measuring lactate dehydrogenase (LDH) activity released in the media 24 h after test compound treatment or glutamate + test compound exposure, using the CytoTox 96 non-radioactive kit and quantitated by measuring wavelength absorbance at 450 nm.

### Results

Following treatment of rat primary cortical cultures with glutamate, there was a significant degeneration of cortical neurons, 24 h post-treatment, demonstrated by an increase in lactate dehydrogenase release into the culture medium.

In primary cortical cultures pre-treated with sarsasapogenin, episarsasapogenin cathylate or smilagenin for 24 h, there was also a significant reduction in the glutamate-induced neurodegeneration (Figure 4)

### Conclusions

Sarsasapogenin, episarsasapogenin cathylate or smilagenin all displayed significant neuroprotective effects against glutamate-induced neurodegeneration in rat primary cortical neurons *in vitro.*

### EXAMPLE 5

### Synthesis of Sarsasapogenin cathylate (3-Ethoxycarbonyl 5β,20α,22α,25S-spirostan-3-β-ol)

Ethyl chloroformate (1.40 g, 12.9 mmol) was added dropwise to a stirred solution of sarsasapogenin (2.08 g, 5.0 mmol) in dry dichloromethane (20 ml) and dry pyridine (1.02 g, 12.9 mmol). The mixture was stirred at room temperature for 18h and then partitioned between water (30 ml) and dichloromethane. The aqueous layer was extracted twice with dichloromethane, the combined organic layers washed with water and then dried over MgSO₄ The solvent was evaporated in vacuo to give an off-white solid (2.6 g). This material was chromatographed on silica using elution with ethyl acetate-hexane (1:9) followed by recrystallisation from acetone (2x) to afford sarsasapogenin cathylate as white crystals (0.72 g, 29%): mp 133-135°C; m/z 488 (M⁺ for C₃₀H₄₈O₅); ¹H NMR (270 MHz, CDCl₃) δ 0.76 (3H, s, 18-CH₃), 0.98 (3H, s, 19-CH₃), 0.99 (3H, d, J = 6.4 Hz, 21-CH₃), 1.08 (3H, d, J = 7.0 Hz, 27-CH₃), 1.09-2.10 (27H, complex m, aliphatic H) overlapping on 1.31 (3H, t, J = 7 Hz, CO₂-C-CH₃), 3.30 (1H, brd, 26-OCHH), 3.95 (1H, brdd, J = 2.7, 10.9 Hz, 26-OCHH), 4.18 (2H, q, J = 7 Hz, CO₂CH₂), 4.40 (1H, brdd, J = 8.8, 7.2 Hz, 16-OCH), 4.95 (1H, br peak, H-3) ppm; ¹³C NMR (67 MHz, CDCl₃) δ 14.3 (C-21, C-C-O₂,C), 16.1, 16.5, 20.9, 23.7, 25.0, 25.8, 26.0, 26.3, 26.4, 27.1, 30.5, 30.6, 31.7, 35.0, 35.3, 37.1, 40.0, 40.3, 40.7, 42.1, 56.4 (C-14), 62.1 (C-17), 63.5 (C-O₂C) 65.1 (C-26), 74.8 (C-3), 81.0 (C-16), 109.7 (C-22), 154.8 (carbonyl) ppm; R_{f} 0.7 (silica, ethyl acetate-hexane, 1:4).

### EXAMPLE 6

### Synthesis of Episarsasapogenin cathylate (3-Ethoxycarbonyl 5β,20α,22α,25S-spirostan-3α-ol)

A solution of lithium tri-tert-butoxyaluminohydride in tetrahydrofuran (1M, 150 ml, 0.15 mol) was carefully added (over 20 min) to a stirred solution of sarsasapogenone (produced by the method of Lajis et al, Steroids, 1993, 58, 387-389) (41.4 g, 0.10 mol) in dry tetrahydrofuran (400 ml) at 20 ± 5° C under dry nitrogen. The reaction mixture was stirred at room temperature for 2h. The resulting solution was carefully quenched with aqueous saturated sodium sulfate solution (50 ml), the inorganic salts removed by filtration through a hyflo pad, and washed with THF. The solvents were removed in vacuo and the residue (ca. 40 g) partitioned between ethyl acetate (500 ml) and 1M hydrochloric acid (200 ml). At the interface of the two solvents a white material remained undissolved that was removed by filtration, washed with water (2 x 100 ml) and dried under vacuum. The solid was slurried twice with ethyl acetate (2 x 250 ml, 5 min each), the solvents decanted off and the insoluble material dried in a vacuum oven. This yielded 23.1 g of crude episarsasapogenin that was recrystallised from acetone (1500 ml) to afford episarsasapogenin as white crystals (12.6 g, 30%): mp 214-216°C; m/z 416 (M⁺ for C₂₇H₄₄O₃); ¹H NMR (270 MHz, CDCl₃) δ 0.75 (3H, s, 18-CH₃), 0.94 (3H, s, 19-CH₃), 0.99 (3H, d, J = 6.6 Hz, 21-CH₃), 1.08 (3H, d, J = 7.3 Hz, 27-CH₃), 1.1-2.1 (27H, complex m, aliphatic H), 3.30 (1H, brd, J = 11.0 Hz, 26-OCHH), 3.55-3.72 (1H, 7 line m, J = 11.0, 5.5, 5.5 Hz, H-3), 3.95 (1H, dd, J = 11.0 , 2.6 Hz, 26-OCHH), 4.40 (1H, dd, J = 8.0, 5.5 Hz, 16-OCH) ppm; ¹³C NMR (67 MHz, CDCl₃) δ 14.3 (C-21), 16.1 (C-27), 16.5 (C-18), 20.6 (C-11), 23.4 (C-19), 25.8 (C-24), 26.0 (C-23), 26.7 (C-6), 27.1 (C-25,C-7), 30.5 (C-2), 31.8 (C-15), 34.7 (C-10), 35.4 (C-1), 35.5 (C-8), 36.5 (C-4), 40.3 (C-12), 40.5 (C-9), 40.6 (C-13), 42.0 (C-5), 42.1 (C-20), 56.4 (C-14), 62.1 (C-17), 65.1 (C-26), 71.8 (C-3), 81.0 (C-16), 109.7 (C-22) ppm; R_{f} 0.35 (silica, ethyl acetate-hexane, 1 :4). A second crop was subsequently obtained (5.2 g). The ethyl acetate extracts from the above experiment were concentrated to ca. 1/5 volume to afford a further crop of episarsasapogenin (3.6 g). Ethyl chloroformate (14.0 g, 0.13 mol) was added dropwise to a stirred solution of episarsasapogenin (1.0.0 g, 0.024 mol) in dry dichloromethane (200 ml) and dry pyridine (10.2 g, 0.13 mol). The pink mixture was stirred at room temperature for 18h and then partitioned between water (30 ml) and dichloromethane. The aqueous layer was extracted twice with dichloromethane, the combined organic layers washed with water and then dried over MgSO₄ The solvent was evaporated in vacuo to afford an off-white solid (13.4 g). Recrystallisation from acetone (ca. 300 ml) yielded episarsasapogenin cathylate as white crystals (8.9 g, 76%); mp 154-156°C; m/z 488 (M⁺ for C₃₀H₄₈O₅); ¹H NMR (270 MHz, CDCl₃) δ 0.75 (3H, s, 18-CH₃), 0.95 (3H, s, 19-CH₃), 0.99 (3H, d, J = 6.6 Hz, 21-CH₃), 1.08 (3H, d, J = 7.0 Hz, 27-CH₃), 1.1-2.1 (27H, complex m, aliphatic H) overlapping with 1.30 (3H, t, J = 7.1 Hz, CO₂-C-CH₃), 3.30 (1H, brd, J = 11.0 Hz, 26-OCHH), 3.95 (1H, dd, J = 11.0, 2.6 Hz, 26-OCHH), 4.18 (2H, q, J = 7 Hz, CO₂CH₂), 4.41 (1H, brdd, J = 8.0, 6.3 Hz, 16-OCH), 4.51-4.66 (1H, 7 line m, H-3) ppm; ¹³C NMR (67 MHz, CDCl₃) δ 14.3 (C-C-O₂C), 14.4 (C-21), 16.1, 16.5, 20.6, 23.3, 25.8, 26.0, 26.5, 26.6, 26.9, 27.1, 31.7, 32.1, 32.8, 34.7, 35.0, 35.4, 40.3, 40.5, 40.6, 41.8, 42.1, 56.4 (C-14), 62.1 (C-17), 63.6 (C-O₂C), 65.1 (C-26), 77.9 (C-3), 81.0 (C-16), 109.7 (C-22), 154.6 (carbonyl) ppm; R_{f} 0.75 (silica, ethyl acetate-hexane, 1:4).

### EXAMPLE 7

### Synthesis of Episarsasapogenin succinate (mono-3α,5β,20α,22α,25S-spirostanyl succinate)

Episarsasapogenin (8.0 g, 19.2 mmol) and succinic anhydride (8.0 g; 80 mmol) were pulverized with a pestle and mortar until a homogeneous mixture of small particle size was obtained. The powdery mixture was then stirred and heated at 80°C on an oil bath while dry pyridine (0.2 ml) was added. The mixture was stirred under nitrogen as the temperature of the bath was raised to 120 ± 5°C to obtain a 'melt', and the melt was maintained at this temperature for 0.5 h. After cooling, the resulting solid was slurried in water (300 ml), acidified with 1M hydrochloric acid and the mixture triturated. The resulting grey tinged solid was collected by filtration, washed with water, dried and recrystallised from methanol (ca. 400 ml), with hot filtration through decolourising charcoal, to afford episarsasapogenin succinate as white crystals (7.46 g, 75%): mp 195-197°C; m/z 516 (M⁺ for C₃₁H₄₈O₆); ¹H NMR (270 MHz, CDCl₃) δ 0.76 (3H, s, 18-CH₃), 0.95 (3H, s, 19-CH₃), 1.00 (3H, d, J = 6.2 Hz, 21-CH₃), 1.08 (3H, d, J = 7.0 Hz, 27-CH₃), 1.2-2.1 (27H, complex m, aliphatic H), 2.63 (4H, m, COCH₂CH₂CO), 3.31 (1H, brd, J = 11.0 Hz, 26-OCHH), 3.96 (1H, dd, J = 11.0, 2.6 Hz, 26-OCHH), 4.42 (1H, brdd, J = 8.0, 6.4 Hz, 16-OCH), 4.75 (1H, m, H-3) ppm; ¹³C NMR (67 MHz, CDCl₃) δ 14.3 (C-21), 16.1 (C-27), 16.5 (C-18), 20.6 (C-11), 23.4 (C-19), 25.8, 25.9, 26.6, 27.0, 27.1, 29.1, 29.3, 31.7 (CCO₂), 32.1 (CCO₂), 34.7, 35.1, 35.5, 40.2, 40.6, 40.7, 41.9, 42.2, 56.3 (C-14), 62.1 (C-17), 65.1 (C-26), 74.9 (C-3), 81.0 (C-16), 109.8 (C-22), 171.7 (ester carbonyl), 177.9 (carboxyl) ppm; R_{f} 0.14 (silica, ethyl acetate-hexane, 3:7).

### EXAMPLE 8

### Synthesis of Epismilagenin cathylate (3-Ethoxycarbonyl 5β,20α,22α,25R-spirostan-3α-ol)

Ethyl chloroformate (1.40g, 12.9 mmol) was added dropwise to a stirred solution of epismilagenin (1.0 g, 2.4 mmol) in dry dichloromethane (30 ml) and dry pyridine (1.02 g, 12.9 mmol). The pink mixture was stirred at room temperature for 4h and then partitioned between water (50 ml) and dichloromethane. The aqueous layer was extracted twice with dichloromethane, the combined organic layers washed with water and then dried over MgSO₄. The solvent was evaporated in vacuo to give a pale yellow solid (1.1 g). Recrystallisation from acetone afforded epismilagenin cathylate as white crystals (0.47 g, 40%); mp 176-179°C; m/z 488 (M⁺ for C₃₀H₄₈O₅) ¹H NMR (270 MHz, CDCl₃) δ 0.75 (3H, s, 18-CH₃), 0.79 (3H, d, J = 6.2 Hz, 27-CH₃), 0.95 (3H, s, 19-CH₃), 0.96 (3H, d, J = 7.3 Hz, 21-CH₃), 1.0-2.05 (27H, complex m, aliphatic H) overlapping with 1.30 (3H, t, J = 7.3 Hz, CO₂-C-CH₃), 3.37 (1H, t, J = 11.0 Hz, 26-OCHH), 3.48 (1H, m, 26-OCHH), 4.17 (2H, q, J = 7.3 Hz, CO₂CH₂), 4.40 (1H, m, 16-OCH), 4.5 8 (1H, 7 line m, H-3) ppm; ¹³C NMR (67 MHz, CDCl₃) δ 14.3 (C-C-O₂C), 14.5 (C-21), 16.5 (C-18), 17.2 (C-27), 20.6 (C-11), 23.3 (C-19), 26.5, 26.6, 26.9, 28.8, 30.3, 31.4, 31.8, 32.1, 34.7, 35.0, 35.4, 40.2, 40.5, 40.6, 41.6, 41.8, 56.4 (C-14), 62.2 (C-17), 63.6 (C-O₂C), 66.8 (C-26), 77.9 (C-3), 80.9 (C-16), 109.2 (C-22), 154.6 (carbonyl) ppm; R_{f} 0.8 (silica, ethyl acetate-hexane, 1:4).

### EXAMPLE 9 (Comparative)

### Synthesis of Episarsasapogenin Glycinate Hydrochloride

### N-tert-butoxycarbonyl 5β,20α,22α,25S-spirostan-3α-yl glycinate (episarsasapogenin BOC glycinate)

Dicyclohexylcarbodiimide (0.68 g, 3.3 mmol) was added in portions over 1 min to a stirred mixture of episarsasapogenin (1.0 g, 2.4 mmol), N-*tert*-butoxycarbonylglycine (0.53 g, 3.0 mmol), 4-dimethylaminopyridine (10 mg, 0.1 mmol) and dry dichloromethane (20ml) at 0-5 °C. The mixture was stirred at room temperature overnight, filtered to remove dicyclohexylurea, and then partitioned between sodium hydrogen carbonate solution (1.5 g in 20 ml water) and dichloromethane (15 ml). The organic layer was separated, washed with 1N hydrochloric acid (15 ml) then water and dried over MgSO₄. The solvent was removed under vacuo to give an off-white foam. This material was triturated and stirred in ether (25 ml) for 3h. After standing overnight the mixture was filtered (to remove any residual dicyclohexylurea) and the filtrate evaporated to give an off-white solid (ca. 1.1 g). Recrystallisation from methanol (ca. 30 ml) afforded the BOC glycinate derivative as white microcrystals (0.40 g): mp 171-173°C; m/z 573.5 (M⁺ for C₃₄H₅₅NO₆) δ _{H} (270 MHz, CDCl₃) 0.76 (3H, s, 18-CH₃), 0.95 (3H, s, 19-CH₃), 0.99 (3H, d, *J* = 6.2 Hz, 21-CH₃), 1.08 (3H, d, *J* = 7.0 Hz 27-CH₃), 1.1-2.1 (27H, complex m, aliphatic H) overlapping with 1.46 (9H, s, C(CH₃)₃), 3.30 (1H, brd, *J*= 11.0 Hz, 26-OC*H*H), 3.86 (2H, brd, *J*= 4.8 Hz, CH₂N). 3.95 (1H, dd, *J*= 11.0, 2.6 Hz, 26-OCH*H*), 4.42 (1H, m, 16-OCH), 4.79 (1H, 7 line m, H-3), 5.04 (1H, brs,NH). δc (270 MHz, CDCl₃) 14.3 (C-21), 16.1 (C-27), 16.5 (C-18), 20.7 (C-11), 23.3 (C-19), 25.8, 26.0, 26.6, 26.9, 27.1, 28.4, 31.8, 32.2, 34.7, 35.0, 35.5, 40.2, 40.6, 40.7, 41.9, 42.2, 42.8, 56.4 (C-14), 62.2 (C-17), 65.2 (C-26), 75.6 (C-3), 79.9 (CH₂N), 81.0 (C-16), 109.7 (C-22), 155.7 (carbamate carbonyl), 169.8 (ester carbonyl) R_{f} 0.4 (silica, ethyl acetate-hexane, 1:8)

### 5β,20α22α,25S-spirostan-3α-yl glycinate Hydrochloride (Episarsasapogenin Glycinate Hydrochloride).

A slow steady stream of hydrogen chloride was passed through a stirred solution of *N-tert-*butoxycarbonyl 5β,20α,22α,25S*-*spirostan*-*3α-yl glycinate (0.40 g, 0.78 mmol) in dry ethyl acetate-ether (24 ml of 1:8) at 0-5 °C with exclusion of moisture. After ca. 45 min the reaction mixture was saturated (excess gas discharging into a trap) and the hydrogen chloride supply disconnected. Stirring was continued and the mixture allowed to warm to room temperature. TLC studies indicated that the deprotection reaction was complete after ca. 2-3h. The resulting white suspension was allowed to stand for 3h, and the powdery white solid removed by filtration and washed with ether. This material was air-dried and then further dried in a vacuum dessicator over CaCl₂ to constant weight to give 0.24 g of a free-flowing white microcrystalline solid, mp 270-272 °C (decomp.) m/z 473 (M⁺ for C₂₉H₄₇NO₄) HCl salt M = 510.2 δ_{H} (270 MHz, CDCl₃) 0.76 (3H, s, 18-CH₃), 0.95 (3H, s, 19-CH₃), 0.99 (3H, d, *J*= 6.2 Hz, 21-CH₃), 1.08 (3H, d, *J*= 7.0 Hz, 27-CH₃), 1.1-2.1 (27H, complex m, aliphatic H) overlapping with 1.46 (9H, s, C(CH₃)₃), 3.30 (1H, brd, *J*= 11.0 Hz, 26-OC*H*H), 3.86 (2H, brd, *J*= 4.8 Hz, CH₂N), 3.95 (1H, dd, *J* = 11.0, 2.6 Hz, 26-OCH*H*), 4.42 (1H, m, 16-OCH), 4.79 (1H, 7 line m, H-3), 5.04 (1H, brs,NH). δc (270 MHz, CDCl₃) 14.3 (C-21), 16.1 (C-27), 16.5 (C-18), 20.7 (C-11), 23.3 (C-19), 25.8, 26.0, 26.6, 26.9, 27.1, 28.4, 31.8, 322, 34.7, 35.0, 35.5, 40.2, 40.6, 40.7, 41.9, 42.2, 42.8, 56.4 (C-14), 62.2 (C-17), 65.2 (C-26), 75.6 (C-3), 79.9 (CH₂N), 81.0 (C-16), 109.7 (C-22), 155.7 (carbamate carbonyl), 169.8 (ester carbonyl) R_{f} 0.6 (silica, dichloromethane-methanol-0.88 ammonia, 9:1:0.1)

### EXAMPLE 10 (Comparative)

### Synthesis of Sarsasapogenin Glycinate Hydrochloride

The title compound was made by a synthesis analogous to that of Example 9, using instead sarsasapogenin, as starting material.

The intermediate, N-tert-butoxycarbonyl 5β, 20α, 22α, 25S-spirostan-3β-yl glycinate (Sarsasapogenin BOC Glycinate) was obtained as white microcrystals, m/z 573.5 (M⁺ for C₃₄H₅₅NO₆) Rᵣ 0.45 (silica, ethyl acetate-hexane, 1:4), and the title compound was obtained as a free-flowing white microcrystalline solid, mp 259-261 °C (decomp.) m/z 473 (M⁺ for C₂₉H₄₇NO₄) HCl salt M = 510.2 R_{f} 0.5 (silica, dichloromethane-methanol-0.88 ammonia, 12:1:0.1)

### EXAMPLE 11 (Comparative)

### Synthesis of Epismilagenin Glycinate Hydrochloride

The title compound was synthesised by a method analogous to that of Example 9, using instead epismilagenin as starting material.

The intermediate, N-tert-butoxycarbonyl 5β,20α,22α,25R-spirostan-3α-yl glycinate (Epismilagenin BOC Glycinate), was obtained as white microcrystals, mp 100-103 **°** C m/z 573.5 (M⁺ for C₃₄H₅₅NO₆) δ_{H} (500 MHz, CDCl₃) 0.75 (3H, s, 18-CH3), 0.79 (3H, d, *J*= 6.3 Hz, 27-CH₃), 0.95 (3H, s, 19-CH₃), 0.96 (3H, d, *J*= 6.9 Hz, 21-CH₃), 1.0-2.0 (27H, complex m, aliphatic H) overlapping with 1.45 (9H, s, C(CH₃)₃), 3.37 (1H, t, *J*= 10.9 Hz, 26-OC*H*H), 3.47 (1H, m, 26-OCH*H*), 3.87 (2H, *J*= 5.3 Hz, CH₂N), 4.40 (1H, m, 16-OCH), 4.79 (1H, 7 line m, H-3), 5.04 (1H, br peak, NH). δc (270 MHz, CDCl₃) 14.7 (C-21), 16.6 (C-18), 17.3 (C-27), 20.8 (C-11), 23.5 (C-19), 26.7, 26.8, 27.1, 28.5, 29.0, 30.5, 31.6, 32.0, 32.3, 34.9, 35.2, 35.6, 40.4, 40.7, 40.8, 41.8, 42.0, 42.9, 56.5 (C-14), 62.4 (C-17), 67.0 (C-26), 75.7 (C-3), 80.1 (CO₂C), 81.1 (C-16), 109.4 (C-22), 155.9 (carbamate carbonyl), 170.0 (ester carbonyl) R_{f} 0.46(silica, ethyl acetate-hexane, 1:4)

The title compound was obtained as a free-flowing white microcrystalline solid, mp 273-275 °C (decomp.) m/z 473 (M⁺ for C₂₉H₄₇NO₄) HCl salt M = 510.2; δ_{H} [500 MHz, (CD₃)₂SO] 0.71 (3H, s, 18-CH₃), 0.75 (3H, d, *J*= 6.3 Hz, 27-CH₃), 0.91 (3H, d, *J*= 6.9 Hz, 21-CH₃), 0.92 (3H, s, 19-CH₃), 1.0-2.0 (27H, complex m, aliphatic H), 3.20 (1H, t, *J*= 11.1 Hz, 26-OC*H*H). 3.41 (1H, m, 26-OCH*H*), 3.71 (2H, brs, CH₂N), 4.28 (1H, m, 16-OCH), 4.75 (1H, 7 line m, H-3), 8.54 (3H, s, NH₃). δc [125 MHz, (CD₃)₂SO] 14.6 (C-21), 16.1 (C-18), 17.0 (C-27), 20.1 (C-11), 22.9 (C-19), 26.0, 26.2, 26.4, 28.5, 29.7, 30.9, 31.4, 31.6, 34.2, 34.3, 34.9, 41.0, 41.1, 55.5 (C-14), 61.9 (C-17), 65.9 (C-26), 75.4 (C-3), 80.2 (C-16), 108.3 (C-22), 166.9 (carbonyl). R_{f} 0.5 (silica, dichloromethane-methanol-0.88 ammonia, 12:1:0.1)

### EXAMPLE 12 (Comparative)

### Synthesis of Epismilagenin L-Alaninate Hydrochloride

The title compound was synthesised by a method analogous to that of Example 9, using instead epismilagenin and N-*tert*-butoxycarbonyl-L-alanine as starting materials.

The intermediate, N-tert-butoxycarbonyl 5β,20α,22α,25R-spirostan-3α-yl L-alaninate (Epismilagenin BOC L-Alaninate), was obtained as white microcrystals, mp 171-173 ° C m/z 587.5 (M⁺ for C₃₅H₅₇NO₆) δ_{H}(500 MHz, CDCl₃) 0.76 (3H, s, 18-CH₃), 0.79 (3H, d, *J* = 6.4 Hz, 27-CH₃), 0.95 (3H, s, 19-CH₃), 0.97 (3H, d, *J* = 7.0 Hz, 21-CH₃), 1.0.2.03 (27H, complex m, aliphatic H) overlapping with 1.37 (3H, d, *J*= 7.1 Hz) and 1.45 (9H, s, C(CH₃)₃), 3.38 (1H, t, *J* = 10.9 Hz, 26-OC*H*H), 3.47 (1H, m, 26-OCH*H*), 4.25 (1H, m, CHN), 4.40 (1H, m, 16-OCH), 4.76 (1H, 7 line m, H-3), 5.06 (1H, br d, *J*= 5.7 Hz, NH). δc (125 MHz, CDCl₃) 14.7 (C-21), 16.6 (C-18), 17.3 (C-27), 19.0 (CH₃-C-N), 20.8 (C-11), 23.5 (C-19), 26.7, 26.8, 27.1, 28.5, 29.0, 30.5, 31.6, 32.0, 32.3, 34.9, 35.2, 35.6, 40.4, 40.7, 40.9, 41.8, 42.0, 49.6, 56.5 (C-14), 62.5 (C-17), 67.0 (C-26), 75.5 (C-3), 79.9 (CO₂C), 81.1 (C-16), 109.4 (C-22), 155.3 (carbamate carbonyl), 173.1 (ester carbonyl) R_{f} 0.53 (silica, ethyl acetate-hexane, 1:4)

The title compound was obtained as a free-flowing white microcrystalline solid, mp 233-235 °C (decomp.) m/z 487 (M⁺ for C₃₀R₄₉NO₄) HCl salt M = 524.2 δ_{H} [500 MHz, (CD₃)₂SO] 0.71 (3H, s, 18-CH₃), 0.74 (3H, d, *J*= 6.3 Hz, 27-CH₃), 0.90 (3H, d, *J*= 6.9 Hz, 21-CH₃), 0.92 (3H, s, 19-CH₃), 1.0-1.95 (27H, complex m, aliphatic H) overlapping with 1.42 (3H, d, *J* = 7.2 Hz, CH₃-C-N), 3.2 (1H, t, *J*= 11.0 Hz, 26-OC*H*H), 3.41 (1H, m, 26-OCH*H*), 3.96 (1H, q, *J*= 7.0 Hz, CHN), 4.29 (1H, m, 16-OCH), 4.73 (1H, 7 line m, H-3), 8.66 (3H, s, NH₃). δc [125 MHz, (CD₃)₂SO] 14.5 (C-21), 15.6 (Ala Me), 16.0 (C-18), 17.0 (C-27), 20.1 (C-11), 22.8 (C-19), 25.9,26.1,26.4,28.4,29.7, 30.9,31.3,31.5, 34.2,34.9, 40.9, 41.0,47.8, 55.5 (C-14), 61.9 (C-17), 65.8 (C-26), 75.4 (C-3), 80.2 (C-16), 108.2 (C-22), 169.3 (carbonyl). Four signals not detected, probably hidden under solvent peaks. R_{f} 0.56 (silica, dichloromethane-methanol-0.88 ammonia, 12:1:0.1)

### EXAMPLE 13 (Comparative)

### Synthesis of Epismilagenin L-Valinate Hydrochloride

The title compound was synthesised by a method analogous to that of Example 9, using instead epismilagenin and N-*tert*-butoxycarbonyl-L-valine as starting materials.

The intermediate, N-tert-butoxycarbonyl 5β,20β,22α,25R-spirostan-3α-yl L-valinate (Epismilagenin BOC L-Valinate) was obtained as white microcrystals, mp 68-71 ° C m/z 615.5 (M⁺ for C₃₇H₆₁NO₆) δ_{H} (500 MHz, CDCl₃) 0.76 (3H, s, 18-CH₃), 0.79 (3H, d, *J*= 6.4 Hz, 27-CH;), 0.89 (6H, d, *J*= 6.9 Hz, C(CH₃)₂), 0.95 (3H, s, 19-CH₃), 0.96 (3H, d, *J*= 6.9 Hz, 21-CH₃), 1.0-2.2 (28H, complex m, aliphatic H) overlapping with 1.43, 1.45 (9H, 2 x s, C(CH₃)₃), 3.38 (1H, t, *J* = 10.9 Hz, 26-OC*H*H), 3.47 (1H, m, 26-OCHH), 4.17 (1H, dd, *J* = 9.9, 4.1 Hz, CHN), 4.40 (1H, m, 16-OCH), 4.79 (1H, 7 line m, H-3), 5.01 (1H, br t, *J* = 9.9 Hz, NH). δc (125 MHz, CDCl₃) 14.7 (C-21), 16.6 (C-18), 17.3 (C-27), 17.7 (Val Me), 19.2 (Val Me), 20.8 (C-11), 23.5 (C-19), 26.7, 26.9, 27.1, 28.5 (t-butyl Me), 29.0, 30.5, 31.6, 32.0, 32.4, 34.9, 35.2, 35.7, 40.4, 40.7, 40.9, 41.8, 42.0, 56.4 (C-14), 58.8 (CHN), 62.5 (C-1 7), 67.1 (C-26), 75.4 (C-3), 79.8 (CO₂C), 81.1 (C-16), 109.4 (C-22), 155.9 (carbamate carbonyl), 172.1 (ester carbonyl) R_{f} 0.60 (silica, ethyl acetate-hexane, 1:4)

The title compound was obtained as a free-flowing white microcrystalline solid, mp 171-173 °C (decomp.) m/z 515.7 (M⁺ for C₃₂H₅₃NO₄) HCl salt M = 552.2; δ_{H} [500 MHz, (CD₃)₂SO] 0.71 (3H, s, 18-CH₃), 0.74 (3H, d, J = 6.4 Hz, 27-CH₃), 0.90 (3H, d, J = 6.9 Hz, 21-CH₃), 0.93 (3H, s, 19-CH₃), 0.95 (3H, d, J = 6.9 Hz, Valine -CH;), 1.00 (3H, d, J = 6.9 Hz, Valine-CH₃), 1.01-2.0 (27H, complex m, aliphatic H), 2.22 (1H, m, CH-C-N), 3.21 (1H, t, J = 11.0 Hz, 26-OCHH), 3.41 (1H, m, 26-OCHH), 3.75 (1H, m, CHN), 4.28 (1H, m, 16-OCH), 4.77 (1H, 7 line m, H-3), 8.6 (3H, br peak, NH₃). δc [125 MHz, (CD₃)₂SO] 14.5 (C-21),16.0 (C-18), 17.0 (C-27), 17.4 (Val Me), 18.4 (Val Me), 20.1 (C-11), 22.9 (C-19), 26.1, 26.2, 26.4, 28.4 (t-Bu), 29.2, 29.7, 30.9, 31.3, 31.7, 34.2, 34.9, 41.0, 41.1, 55.5 (C-14), 57.1 (CHN), 61.9 (C-17), 65.8 (C-26), 75.4 (C-3), 80.2 (C-16), 108.3 (C-22), 168.0 (carbonyl). Four signals not detected, probably hidden under solvent peaks. R_{f} 0.64 (silica, dichloromethane-methanol-0.88 ammonia, 12:1:0.1)

### EXAMPLE 14 (Comparative)

### Synthesis of Epismilagenin L-Isoleucinate Hydrochloride

The title compound was synthesised by a method analogous to that of Example 9, using instead epismilagenin and N-tert-butoxycarbonyl-L-isoleucine as starting materials.

The intermediate, N-tert-butoxycarbonyl 5β,20α,22α,25R spirostan-3α-yl L-isoleucinate (Epismilagenin BOC L-Isoleucinate), was obtained as white microcrystals, mp 67-70 ° C m/z 629.5 (M⁺ for C₃₈H₆₃NO₆) R_{f} 0.6 (silica, ethyl acetate-hexane, 1:4)

The title compound was obtained as a free-flowing white microcrystalline solid, mp 169-171 °C (decomp.) m/z 529.7 (M⁺ for C₃₃H₅₅NO₄) HCl salt M = 566.2 R_{f} 0.7 (silica, dichloromethane-methanol-0.88 ammonia, 12:1:0.1)

### EXAMPLE 15 (Comparative)

### Synthesis of Epismilagenin L-Phenylalaninate Hydrochloride

The title compound was synthesised by a method analogous to that of Example 9, using instead epismilagenin and N-*tert*-butoxycarbonyl-L-phenylalanine as starting materials.

The intermediate, N-tert-butoxycarbonyl 5β,20α,22α,25R-spirostan-3α-yl L-phenylalaninate (Epismilagenin BOC L-Phenylalaninate), was obtained as white microcrystals, mp 66-68 ° C m/z 663.5 (M⁺ for C₄,H₆,NO₆) R_{f} 0.6 (silica, ethyl acetate-hexane, 1:5).

The title compound was obtained as a free-flowing white microcrystalline solid, mp 254-256 °C (decomp.) m/z 563.5 (M⁺ for C₃₆H₅₃NO₄) HCl salt M = 600.2 R_{f} 0.6 (silica, dichloromethane-methanol-0.88 ammonia, 12:1:0.1)

### EXAMPLE 16 (Comparative)

### Synthesis of Epismilagenin L-Methioninate Hydrochloride

The title compound was synthesised by a method analogous to that of Example 9, using instead epismilagenin and N-*tert*-butoxycarbonyl-L-methionine as starting materials.

The intermediate, N-tert-butoxycarbonyl 5β,20α,22α,25R-spirostan-3α-yl L-methioninate (Epismilagenin BOC L-Methioninate), was obtained as white microcrystals, mp 76-79 ° C m/z 647.9 (M⁺ for C₃₇H₆₁NO₆ S) R_{f} 0.5 (silica, ethyl acetate-hexane, 1:5)

The title compound was obtained as a free-flowing white microcrystalline solid, mp 173-176 °C (decomp.) m/z 547.8 (M⁺ for C₃₂H₅₃NO₄S) HCl salt M = 584.3 R_{f} 0.5 (silica, dichloromethane-methanol-0.88 ammonia, 12:1:0.1)

## Claims

1. Compounds of the general formula II: 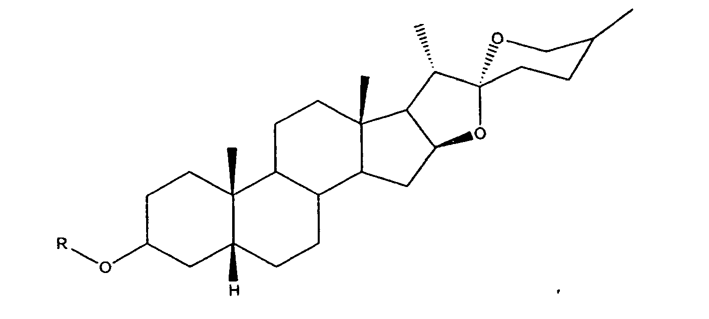 wherein the group R is selected from (C₁₋₄ alkyl)carbonyl and (C₁₋₄ alkoxy)carbonyl, optionally substituted with a terminal carboxylic acid residue (-COOH);
provided that:
R is not unsubstituted acetyl;
R is not unsubstituted ethoxycarbonyl when simultaneously the stereochemistry of C3 is S(β) and of C25 is R;
R is not succinyl when simultaneously the stereochemistry of C3 is S(β) and of C25 is S or the stereochemistry of C3 is R(α) or S(β) and of C25 is R; and
R is not propionyl, butyryl, isobutyryl, valeryl or isovaleryl when the stereochemistry of C25 is R and the stereochemistry of C3 is S(β);
and pharmaceutically acceptable salts thereof.

2. Compounds as claimed in claim 1, wherein the C₂₅ methyl group is in the R configuration.

3. Compounds as claimed in claim 1, wherein the C₂₅ methyl group is in the S configuration.

4. A compound as claimed in claim 1 selected from epismilagenin cathylate, sarsasapogenin cathylate, episarsasapogenin cathylate, and episarsasapogenin succinate.

5. Compounds according to any one of claims 1 to 4, for use in the treatment of one of: Parkinson's disease, postural hypotension, autism, chronic fatigue syndrome, Myasthenia Gravis, Lambert Eaton disease, Huntington disease, multiple sclerosis, asthma, heart failure and epilepsy.

6. A pharmaceutical composition which comprises a pharmacologically effective amount of a compound as claimed in any one of claims 1 to 4.

7. A foodstuff or beverage which comprises a pharmacologically effective amount of a compound as claimed in any one of claims 1 to 4.

## Patentansprüche

1. Verbindungen der Formel II: 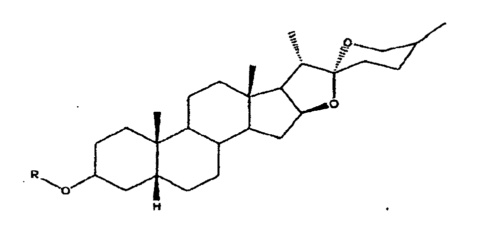 in der die Gruppe R aus (C₁₋₄Alkyl)carbonyl und (C₁₋₄Alkoxy)carbonyl, optional mit einem endständigen Karboxylsäurerest (-COOH) substituiert, ausgewählt wurde,
insofern:
R nicht unsubstituiertes Azetyl ist,
R nicht unsubstituiertes Ethoxycarbonyl ist, wenn gleichzeitig die Stereochemie von C3 S(β) ist und die von C25 R ist,
R nicht Sukzinyl ist, wenn gleichzeitig die Stereochemie von C3 S(β) ist und die von C25 R ist oder die Stereochemie von C3 R(α) ist und die von C25 R ist,
R nicht Propionyl, Butyryl, lsobutyryl, Valeryl oder Isovaleryl ist, wenn die Stereochemie von C25 R ist und die Stereochemie von C3 S(β) ist,
und pharmazeutisch anwendbare Salze davon.

2. Verbindungen nach Patentanspruch 1, in denen sich die C₂₅-Methylgruppe in der R-Konfiguration befindet.

3. Verbindungen nach Patentanspruch 1, in denen sich die C₂₅-Methylgruppe in der S-Konfiguration befindet.

4. Verbindung nach Patentanspruch 1, ausgewählt unter Epi-Smilagenincathylat, Sarsasapogenin-cathylat, Epi-Sarsasapogenin-cathylat, und Epi-Sarsasapogenin-Sukzinat.

5. Verbindungen nach irgendeinem der Patentansprüche 1 bis 4 zum Gebrauch in der Behandlung von: Parkinson-Krankheit, posturaler Hypotonie, Autismus, chronischem Erschöpfungssyndrom, Myasthenia gravis, Lambert-Eaton-Syndrom, Chorea Huntington, Multiple Sklerose, Asthma, Herzversagen und Epilepsie.

6. Pharmazeutische Zubereitung, die eine pharmakologisch wirksame Menge einer Verbindung enthält, wie sie in irgendeinem der Patentansprüche 1 bis 4 beansprucht wird.

7. Lebensmittel oder Getränk, das eine pharmakologisch wirksame Menge einer Verbindung enthält, wie sie in irgendeinem der Patentansprüche 1 bis 4 beansprucht wird.

## Revendications

1. Composés de formule générale II : 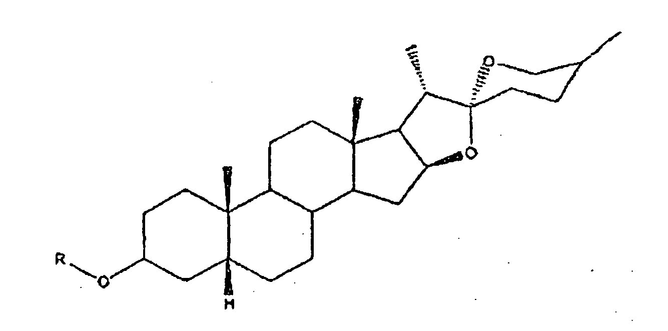 dans laquelle le groupe R est choisi parmi alcoylcarbonyle ayant de 1 à 4 atomes de carbone dans la partie alcoyle et alcoxycarbonyle ayant de 1 à 4 atomes de carbone dans la partie alcoxy, substitué éventuellement par un radical acide carboxylique d'extrémité (-COOH) ;
pourvu que:
R ne soit pas acétyle non substitué ;
R ne soit pas éthoxycarbonyle non substitué lorsque simultanément la stéréochimie de C3 est S(β) et celle de C25 est R ;
R ne soit pas succinyle lorsque simultanément la stéréochimie de C3 est S(β) et celle de C25 est S, ou la stéréochimie de C3 est R(α) ou S(β) et celle de C25 est R ; et
R ne soit pas propionyle, butyryle, isobutyryle, valéryle ou isovaléryle lorsque la stéréochimie de C25 est R et lorsque la stéréochimie de C3 est S(β) ;
et leurs sels acceptables pharmaceutiquement.

2. Composés suivant la revendication 1, dans lesquels le groupe méthyle en C₂₅ est dans la configuration R.

3. Composés suivant la revendication 1, dans lesquels le groupe méthyle en C₂₅ est dans la configuration S.

4. Composé suivant la revendication 1, choisi parmi le cathylate d'épismilagénine, le cathylate de sarsasapogénine, le cathylate d'épisarsasapogénine et le succinate d'épisarsasapogénine.

5. Composés suivant l'une quelconque des revendications 1 à 4, à utiliser dans le traitement de l'un de : maladie de Parkinson, hypotension orthostatique, autisme, syndrome de fatigue chronique, myasthénie grave, maladie de Lambert Eaton, maladie d'Huntington, sclérose en plaques, asthme, insuffisance cardiaque et épilepsie.

6. Composition pharmaceutique qui comprend une quantité efficace pharmacologiquement d'un composé tel que revendiqué dans l'une quelconque des revendications 1 à 4.

7. Denrée alimentaire ou boisson qui comprend une quantité efficace pharmacologiquement d'un composé tel que revendiqué dans l'une quelconque des revendications 1 à 4.
